# EUROPEAN PATENT SPECIFICATION

(11) **EP 2 078 058 B1**
(45) Date of publication and mention of the grant of the patent: **16.06.2010**
(21) Application number: 07788143.1
(22) Date of filing: 01.08.2007
(51) Int. Cl.: C09C 1/04

(54) **SURFACE-MODIFIED ZINC OXIDE-SILICON DIOXIDE CORE-SHELL PARTICLES**
OBERFLÄCHLICH MODIFIZIERTE ZINKOXID-SILICIUMDIOXID-KERN-SCHALE-PARTIKEL
PARTICULES C UR-ÉCORCE D'OXYDE DE ZINC-DIOXYDE DE SILICIUM MODIFIÉES EN SURFACE

(30) Priority: 02.11.2006 DE 102006051634
(43) Date of publication of application: 15.07.2009
(73) Proprietor: Evonik Degussa GmbH, 45128 Essen (DE)
(72) Inventor: KATUSIC, Stipan, 65812 Bad Soden (DE); ZIMMERMANN, Guido, 50321 Brühl (DE); MEYER, Jürgen, 63811 Stockstadt (DE)
(86) International application number: PCT/EP2007/057978
(87) International publication number: WO 2008/052817

(56) References cited:
- WO-A-2005/071002
- WO-A-2005/075579
- US-B1- 6 534 044

## Description

The invention relates to surface-modified zinc-silicon oxide particles, and to their preparation and use.

It is known, for reducing the photocatalytic activity of UV-absorbing substances, to provide them with an inert coating. The UV-absorbing substances may be organic or inorganic substances. Of particular importance are the titanium dioxides and zinc oxides coated with silicon dioxide, which are used primarily in sun protection formulations. A disadvantage of these coated products is their inadequate dispersibility in media containing cosmetic, organic constituents.

In US 6534044, this problem is solved through the use of hydrophobic zinc oxide particles coated with silicon dioxide. The coated particles are obtained by adding to an aqueous-alcoholic dispersion containing the zinc oxide particles a tetraalkoxysilane which is hydrolysed in the present medium to silicon dioxide. Subsequently, the zinc oxide particles coated with silicon dioxide are provided with a hydrophobic coating. The hydrophobic coating improves the incorporability into organic media compared with nonhydrophobicized particles. This improved incorporability finds its limits in the high degree of intergrowth of the hydrophobic zinc oxide particles coated with silicon dioxide disclosed in US 6534044.

WO2005/075579 discloses surface modified coated metal oxide particles with a low structure, consisting of a core of a metal oxide and a shell of silicon oxide surrounding the core.

WO2005/071002 discloses a dispersion comprising a ZnO-powder, surface-modified by silanes.

It was therefore an object of the invention to provide particles with high UV absorption with simultaneous low photocatalytic activity which have an improved incorporability, compared with the prior art, in cosmetic media containing organic constituents.

The object is achieved by surface-modified zinc-silicon oxide particles which consist of a core, a first coating layer surrounding the core, and a second coating layer surrounding the first coating layer, where
- the core is crystalline and consists of aggregated zinc oxide primary particles having a primary particle diameter of from 10 to 75 nm,
- the first coating layer consists of one or more compounds containing the elements Zn, Si and O,
- the second coating layer comprises linear and/or branched monoalkylsilyl groups Si-alkyl and/or dialkylsilyl groups Si-(alkyl)₂ having 1 to 20 carbon atoms bonded chemically to the first coating layer,
- their average aggregate area is less than 40 000 nm² and their average aggregate diameter (ECD) is less than 300 nm,
- their carbon content is 0.4 to 1.5% by weight and
- their BET surface area is 10 to 60 m²/g.

The primary particle diameter of the zinc oxide core is 10 to 75 nm, preferably 20 to 50 nm, and is determined by means of image analysis. The distances between the lattice planes, determined from HR-transmission electron micrographs, show that the core consists of crystalline zinc oxide.

The average aggregate area of the surface-modified zinc-silicon oxide particles according to the invention is likewise determined by image analysis. These are characterized by a small average aggregate area of less than 40 000 nm². In one preferred embodiment, the average aggregate area is less than 20 000 nm², where a range from 1000 to 10 000 nm² may be particularly preferred.

The average aggregate diameter (ECD) of the surface-modified zinc-silicon oxide particles according to the invention likewise determined by means of image analysis is less than 300 nm. Preferably, the average aggregate diameter (ECD) is 100 to 200 nm.

The first coating layer comprises, according to XPS-ESCA analysis (XPS = X-ray photoelectron spectroscopy; ESCA = electron spectroscopy for chemical analysis) and TEM-EDX analysis (transmission electron microscopy [TEM] in conjunction with energy-dispersive analysis of characteristic X-rays [EDX]), compounds which contain the elements Si and O. Furthermore, the coating can also have compounds containing zinc.

The amount of these elements and of the corresponding compounds in the first coating layer cannot be determined exactly. However, evaluation of TEM-EDX and XPS-ESCA spectra clearly shows that Si and O are the main components of the coating, and Zn, if present, is only present in secondary amounts. The presence of zinc in the first coating layer does not change the properties of the surface-modified zinc-silicon oxide particles according to the invention.

The first coating layer is preferably in amorphous form. The coating can furthermore have small crystalline fractions detectable by X-ray diffractometry. The fractions are usually barely above the detection limit in the X-ray diffractometry.

The statement that the first coating layer can have crystalline constituents is based firstly on the evaluation of the lattice distances in HR-transmission electron micrographs, which clearly reveals the core to be zinc oxide, and secondly on the fact that the X-ray diffractogram shows further signals of low intensity apart from zinc oxide. The assignment of these signals to compounds is currently not possible.

The thickness of the first coating layer is not limited. A thicker coating layer is favourable for reducing the photocatalytic activity, but unfavourable for the UV absorption of the particles. A thickness of the first coating layer of from 0.1 to 10 nm leads to particularly favourable values for UV absorption and photocatalytic activity and is therefore preferred, for example, for applications in the field of sun protection formulations. Very particular preference is given to a range from 1 to 5 nm.

The second coating layer comprises linear and/or branched alkylsilyl groups having 1 to 20 carbon atoms bonded chemically to the first coating layer.

The structures A-J depict possible alkylsilyl groups according to the invention. Here, the oxygen atom of the -O-Si bond in each case represents and oxygen atom of the surface of the first coating layer.

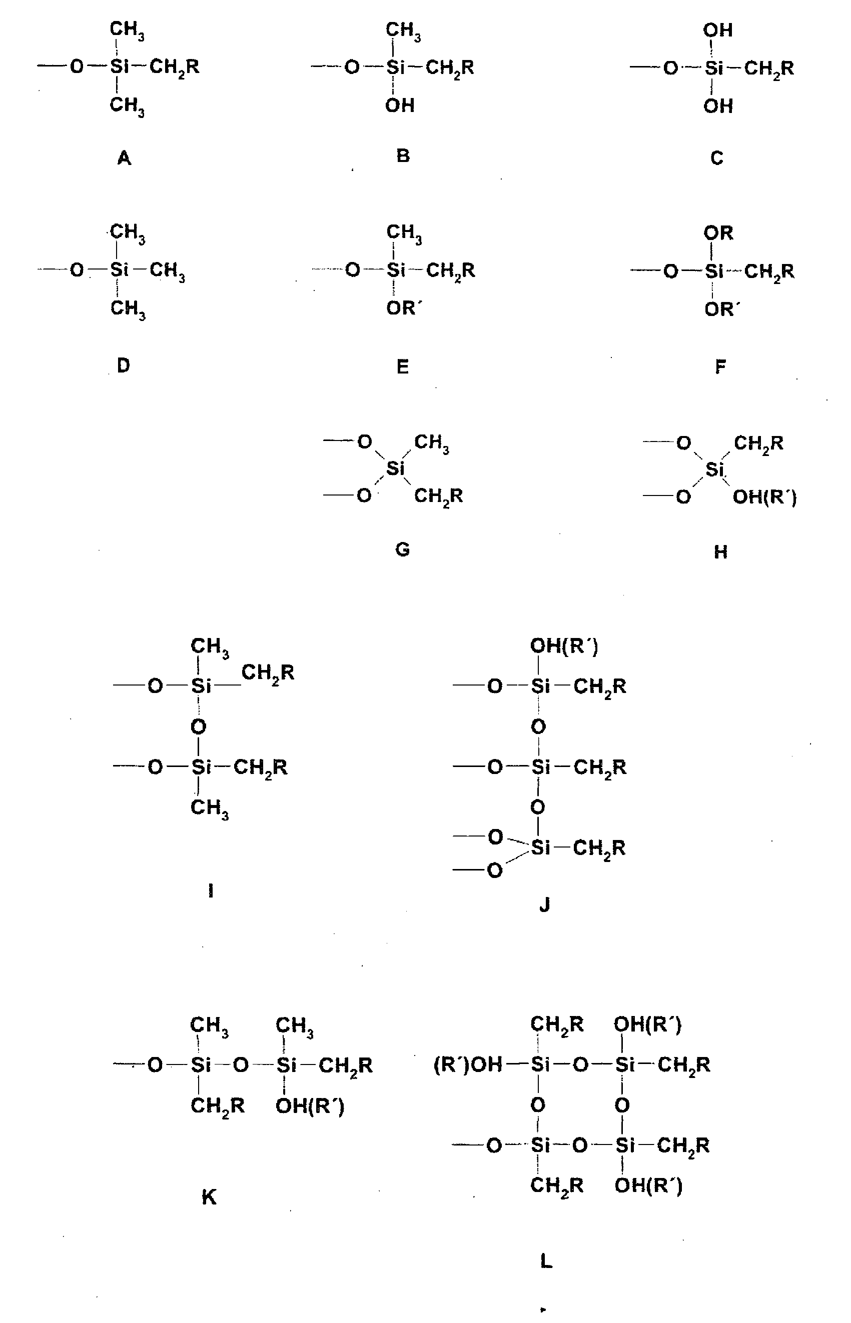

Particular preference may be given to surface-modified zinc-silicon oxide particles in which
- the second coating layer consists of alkylsilyl groups having 1 to 8 carbon atoms,
- the carbon content is from 0.8 to 1.2% by weight,
- the BET surface area is 20 to 40 m²/g
- the average aggregate area is less than 20 000 nm² and the average aggregate diameter is less than 150 nm.

The invention further provides a process for the preparation of the surface-modified zinc-silicon oxide particles according to the invention in which zinc-silicon oxide particles are sprayed with one or more surface-modifying agents, optionally dissolved in an organic solvent, selected from the group comprising silanes, polysiloxanes and/or silazanes having in each case linear and/or branched C₁-C₂₀-alkyl units, and the mixture is then thermally treated at a temperature of from 120 to 200°C over a period of from 0.5 to 2 hours, optionally under protective gas, where the zinc-silicon oxide particles used
- have a Zn/Si ratio of from 2 to 75, in atom%/atom%,
- have a proportion of Zn, Si and O of at least 99% by weight, based on the zinc-silicon oxide particles,
- have a BET surface area of from 10 to 60 m²/g, a weight-averaged primary particle diameter of from 10 to 75 nm, an average aggregate area of less than 40 000 nm² and an average aggregate diameter (ECD) of less than 200 nm,
- consist of a crystalline core of aggregated primary particles of zinc oxide and of a coating which surrounds the aggregated zinc oxide primary particles and of one or more compounds containing the elements Si and O.

The zinc-silicon oxide particles used are subject matter of the as yet unpublished German patent application with the application number 102006038518.7 and 17 August 2006 as filing date.

The zinc-silicon oxide particles used can also have the following features:
- the coating can consist of one or more compounds containing the elements Si, O and Zn.
- the Zn/Si ratio is 3 to 15.
- the BET surface area is 20 to 40 m²/g.
- the average aggregate area is less than 20 000 nm² and the average aggregate diameter is less than 150 nm.
- the thickness of the coating is 0.1 to 10 nm.
- the maximum extinction/extinction at 450 nm ratio is 4 to 8.
- the photocatalytic activity, expressed by the photon efficiency and determined by the degradation of dichloroacetic acid, is less than 0.4.
- upon heating to 1400°C, they lose less than 2% of their mass.
- they comprise at most 20 ppm of Pb, at most 3 ppm of As, at most 15 ppm of Cd, at most 200 ppm of Fe, at most 1 ppm of Sb and at most 1 ppm of Hg.

Silanizing agents which can be used are preferably
- haloorganosilanes of the type X₃Si(CₙH₂ₙ₊₁), X₂(R')Si(CₙH₂ₙ₊₁), X(R')₂Si(CₙH₂ₙ₊₁), X₃Si(CH₂)ₘ-R', (R)X₂Si(CH₂)ₘ-R', (R)₂XSi(CH₂)ₘ-R' where X = Cl, Br; R = alkyl; R' = alkyl; n = 1-20; m = 1-20;
- organosilanes of the type (RO)₃Si(CₙH₂ₙ₊₁), R'ₓ(RO)_{y}Si(CₙH₂ₙ₊₁), (RO)₃Si(CH₂)ₘ-R', (R")u(RO)vSi(CH₂)ₘ-R' where R = alkyl; R' = alkyl; n = 1-20; m = 1-20; x + y = 3; x = 1, 2; y = 1, 2; u + v = 2; u = 1 2; v = 1, 2;
- silazanes of the type R'R₂Si-NH-SiR₂R' where R = alkyl, R' = alkyl, vinyl;
- polysiloxanes of the type 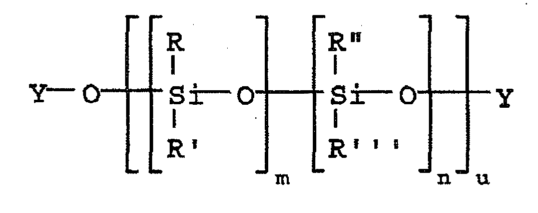 where R = alkyl, H; R' = alkyl, H; R" = alkyl, H; R"' = alkyl, H; Y = CH₃, H, CₚH₂ₚ₊₁ where p = 1-20; Y = Si(CH₃)₃, Si(CH₃)₂H, Si(CH₃)₂OH, Si(CH₃)₂(OCH₃), Si(CH₃)₂(CₚH₂ₚ₊₁) where p = 1-20; m = 0, 1, 2, 3, ...∞; n = 0, 1, 2, 3, ...∞; u = 0, 1, 2, 3, ...∞ or
- cyclic polysiloxanes of the type D3, D4 and/or D5.

The use of trimethoxyoctylsilane [(CH3O)-Si-C8H17], for example DYNASYLAN^{®} OCTMO, Degussa AG, hexamethyldisilazane, for example DYNASYLAN^{®} HMDS, Degussa AG or polydimethylsiloxane as silanizing agent may be particularly preferred.

Furthermore, the process according to the invention can be carried out continuously or discontinuously in heatable mixers and dryers with spray devices, for example in ploughshare mixers, disc dryers, fluidized-bed dryers or moving-bed dryers.

A dispersion which comprises the surface-modified zinc-silicon oxide particles according to the invention is further provided by the invention.

The liquid phase of the dispersion can be water, one or more organic solvents or an aqueous/organic combination, where the phases are miscible.

Liquid, organic phases may be, in particular, methanol, ethanol, n-propanol and isopropanol, butanol, octanol, cyclohexanol, acetone, butanone, cyclohexanone, ethyl acetate, glycol ester, diethyl ether, dibutyl ether, anisole, dioxane, tetrahydrofuran, mono-, di-, tri- and polyglycol ether, ethylene glycol, diethylene glycol, propylene glycol, dimethylacetamide, dimethylformamide, pyridine, N-methylpyrrolidine, acetonitrile, sulpholane, dimethyl sulphoxide, nitrobenzene, dichloromethane, chloroform, tetrachloromethane, ethylene chloride, pentane, hexane, heptane and octane, cyclohexane, benzines, petroleum ether, methylcyclohexane, decalin, benzene, toluene and xylenes. Ethanol, n- and isopropanol, ethylene glycol, hexane, heptane, toluene and o-, m- and p-xylene are particularly preferred as organic, liquid phase.

The dispersion according to the invention can further comprise pH regulators, surface-active additives and/or preservatives.

The content of hydrophobic zinc oxide particles according to the invention can preferably be 0.5 to 60% by weight. Particular preference is given to a dispersion comprising 10 to 50% by weight, in particular 35 to 45% by weight, of the hydrophobic zinc oxide particles according to the invention.

The average particle size in the dispersion can be varied within a wide range using appropriate dispersion units. These may, for example, be rotor-stator machines, high-energy mills, in which the particles grind themselves through collision with one another, planetary kneaders, stirred ball mills, ball mills operating as shaking unit, shaking panels, ultrasound units or combinations of the abovementioned units.

A particularly small particle size can be obtained by using rotor-stator machines and high-energy mills. The average particle size d₅₀ can here assume values of less than 180 nm, in particular less than 140 nm, determined by means of dynamic light scattering.

The invention further provides a sun protection formulation which comprises the hydrophobic zinc oxide particles according to the invention or the dispersion according to the invention.

These are present in the sun protection formulation usually in an amount of from 0.5 to 20% by weight, preferably 1 to 10% by weight and particularly preferably 3 to 8% by weight.

The sun protection formulation according to the invention can also comprise all water-soluble or oil-soluble UVA and UV-B filters known to the person skilled in the art.

For example
- paraaminobenzoic acid (PABA) and derivatives thereof, such as dimethyl-, ethyldihydroxypropyl-, ethylhexyldimethyl-, ethyl-, glyceryl- and 4-bis(polyethoxy)-PABA.
- cinnamic acid esters, such as methyl cinnamate and methoxycinnamic acid esters, comprising octyl methoxycinnamate, ethyl methoxycinnamate, 2-ethylhexyl p-methoxycinnamate, isoamyl p-methoxycinnamate, diisopropyl cinnamate, 2-ethoxyethyl 4-methoxycinnamate, DEA methoxycinnamate (diethanolamine salt of p-methoxyhydroxycinnamic acid ester), diisopropyl methyl cinnamate;
- benzophenones, such as 2,4-dihydroxy-, 2-hydroxy-4-methoxy-, 2,2'-dihydroxy-4,4'-dimethoxy-, 2,2'-dihydroxy-4-methoxy-, 2,2',4,4'-tetrahydroxy-, 2-hydroxy-4-methoxy-4'-methylbenzophenones, sodium 2,2'-dihydroxy-4,4'-dimethoxy-5-sulphobenzophenones.
- dibenzoylmethanes, such as butylmethoxydibenzoylmethane, in particular 4-tert-butyl-4'-methoxydibenzoylmethane;
- 2-phenylbenzimidazole-5-sulphonic acid and phenyldibenzimidazolesulphonic acid esters and salts thereof;
- diphenylacrylates, such as alkyl alpha-cyano-beta,beta-diphenylacrylates, such as octocrylene;
- triazines, such as 2,4,6-trianiline(p-carbo-2-ethylhexyl-1-oxy)-1,3,5-triazine, ethylhexyltriazone and diethylhexylbutamidotriazone.
- camphor derivatives, such as 4-methylbenzylidene- and 3-benzylidenecamphor and terephthalylidene-dicamphorsulphonic acid, benzylidenecamphorsulphonic acid, camphorbenzalkonium methosulphate and polyacrylamidomethylbenzylidenecamphor;
- salicylates, such as dipropylene glycol, ethylene glycol, ethylhexyl, isopropylbenzyl, methyl, phenyl, 3,3,5-trimethyl and TEA salicylates (compound of 2-hydroxybenzoic acid and 2,2',2"-nitrilotrisethanol);
- esters of 2-aminobenzoic acid.

The sun protection formulation can further comprise compounds known to the person skilled in the art, such as organic solvents, thickeners, emulsifiers, softeners, antifoams, antioxidants, plant extracts, moisturizing agents, perfumes, preservatives and/or dyes, complexing agents, anionic, cationic, nonionic or amphoteric polymers or mixtures thereof, propellant gases and finely divided powders, including metal oxide pigments with a particle size of from 100 nm to 20 µm.

Suitable softeners are, in particular, avocado oil, cottonseed oil, behenyl alcohol, butyl myristate, butyl stearate, cetyl alcohol, cetyl palmitate, decyl oleate, dimethylpolysiloxane, di-n-butyl sebacate, thistle oil, eicosanyl alcohol, glyceryl monoricinoleate, hexyl laurate, isobutyl palmitate, isocetyl alcohol, isocetyl stearate, isopropyl isostearate, isopropyl laurate, isopropyl linoleate, isopropyl myristate, isopropyl palmitate, isopropyl stearate, isostearic acid, cocoa butter, coconut oil, lanolin, lauryl lactate, corn oil, myristyl lactate, myristyl myristate, evening primrose oil, octadecan-2-ol, olive oil, palmitic acid, palm kernel oil, polyethylene glycol, rapeseed oil, castor oil, sesame oil, soya oil, sunflower oil, stearic acid, stearyl alcohol, triethylene glycol.

Suitable emulsifiers are, in particular, glycerol monolaurate, glycerol monooleate, glycerol monostearate, PEG 1000 dilaurate, PEG 1500 dioleate, PEG 200 dilaurate, PEG 200 monostearate, PEG 300 monooleate, PEG 400 dioleate, PEG 400 monooleate, PEG 400 monostearate, PEG 4000 monostearate, PEG 600 monooleate, polyoxyethylene(4) sorbitol monostearate, polyoxyethylene(10) cetyl ether, polyoxyethylene(10) monooleate, polyoxyethylene(10) stearyl ether, polyoxyethylene(12) lauryl ether, polyoxyethylene(14) laurate, polyoxyethylene(2) stearyl ether, polyoxyethylene(20) cetyl ether, polyoxyethylene(20) sorbitol monolaurate, polyoxyethylene(20) sorbitol monooleate, polyoxyethylene(20) sorbitol monopalmitate, polyoxyethylene(20) sorbitol monostearate, polyoxyethylene(20) sorbitol trioleate, polyoxyethylene(20) sorbitol tristearate, polyoxyethylene(20) stearyl ether, polyoxyethylene(23) lauryl ether, polyoxyethylene(25) oxypropylene monostearate, polyoxyethylene(3.5) nonylphenol, polyoxyethylene(4) lauryl ether, polyoxyethylene(4) sorbitol monolaurate, polyoxyethylene(5) monostearate, polyoxyethylene(5) sorbitol monooleate, polyoxyethylene(50) monostearate, polyoxyethylene(8) monostearate, polyoxyethylene(9.3) octylphenol, polyoxyethylene sorbitol lanolin derivatives, sorbitol monolaurate, sorbitol monooleate, sorbitol monopalmitate, sorbitol monostearate, sorbitol sesquioleate, sorbitol tristearate, sorbitol trioleate.

Suitable propellant gases may be propane, butane, isobutane, dimethyl ether and/or carbon dioxide.

Suitable finely divided powders may be chalk, talc, kaolin, colloidal silicon dioxide, sodium polyacrylate, tetraalkyl- and/or trialkylarylammonium smectites, magnesium aluminium silicates, montmorillonite, aluminium silicates, fumed silicon dioxide, fumed titanium dioxide.

Typically, the sun protection composition according to the invention can be in the form of an emulsion (O/W, W/O or multiple), aqueous or aqueous-alcoholic gel or oil gel, and be supplied in the form of lotions, creams, milk sprays, mousse, stick or in other customary forms.

### Examples:

### Analytical methods

The BET surface area is determined in accordance with DIN 66131.

The transmission electron micrographs are obtained using a Hitachi TEM instrument, model H-75000-2. Using the CCD camera of the TEM instrument and subsequent image analysis, about 1000 to 2000 aggregates are evaluated. The definition of the parameters is in accordance with ASTM 3849-89. The shape analysis of the aggregates as circular, ellipsoidal, linear and branched is carried out in accordance with Herd et al., Rubber, Chem. Technol. 66 (1993) 491.

Example 1: 3 kg/h of zinc powder (particle diameter d₅₀ = 25 µm) are transferred by means of a nitrogen stream (15 Nm³/h) into a vaporization zone, where a hydrogen/air flame, hydrogen 14.5 Nm³/h, air 30 Nm³/h, burns. The zinc is vaporized.

Vaporization zone conditions: lambda: 0.80, average residence time: 1009 ms, temperature: 1080°C.

Afterwards, 65 Nm³/h of oxidation air are added to the reaction mixture and then 2.45 kg/h of TEOS are introduced into the oxidation zone by means of 4 Nm³/h of atomization air.

Zinc oxidation zone conditions: lambda: 8.76; average residence time: 29 ms, temperature: 800°C.

Silicon oxidation zone conditions: lambda: 4.04; average residence time: 51 ms, temperature: 760°C.

For cooling the hot reaction mixture, 200 Nm³/h of quenching air are added. The powder obtained is then separated off from the gas stream by filtration.

The powder has the physicochemical values given in Table 2.

Examples 2 and 3 are carried out analogously to Example 1. Feed materials and amounts used are given in Table 1. The physicochemical values are given in Table 2.

### Preparation of surface-modified zinc-silicon oxide particles according to the invention

Zinc-silicon oxide particles 1A are initially introduced into a mixer. With intense mixing, they are firstly optionally sprayed with water and then sprayed with a surface-modifying agent. When the spraying operation is complete, they are after-mixed for about a further 15 minutes and then heat-treated.
The zinc-silicon oxide particles 2A and 3A are converted analogously. Feed materials and reaction conditions are given in Table 3.

### Sun protection formulations

The sun protection formulations according to the invention which, in the combination of surface-modified zinc-silicon oxide particles from Example 1B, have shown a synergistic effect with either OC = octocrylene, OMC = ethylhexyl methoxycinnamate, ISA = phenylbenzimidazole sulphonic acid or BEMT = bisethylhexyloxymethoxyphenyltriazine, are listed below.

The SPF (sun protection factor) measurements are carried out in vitro using an Optometrics SPF 290-S instrument. The results show that the surface-modified zinc-silicon oxide particles according to the invention can be incorporated into cosmetic formulations in an excellent manner.

### Examples 4A-C (Table 5)

In these examples, the standard formulation for W/O emulsions is used. The Zn-Si oxide particles from Example 1B are introduced into the oil phase of the system.

| | |
|---|---|
| A | standard formulation W/O emulsion with Zn-Si oxide |
| B | standard formulation W/O emulsion with OC |
| C | standard formulation W/O emulsion with Zn-Si oxide and OC |

### Examples 5A-D (Table 6)

In these examples, the standard formulation for O/W emulsions is used. The Zn-Si oxide particles from Example 1B are, introduced into the oil phase of the system.

| | |
|---|---|
| A | standard formulation O/W emulsion with Zn-Si oxide |
| B | standard formulation O/W emulsion with OC |
| C | standard formulation O/W emulsion with Zn-Si oxide |
| D | standard formulation O/W emulsion with Zn-Si oxide + isostearic acid |

### Examples 6A-C (Table 7)

Tn these examples, the standard formulation for W/O emulsions is used. The Zn-Si oxide particles from Example 1B are introduced into the oil phase of the system.

| | |
|---|---|
| A | standard formulation W/O emulsion with Zn-Si oxide |
| B | standard formulation W/O emulsion with OMC |
| C | standard formulation W/O emulsion with Zn-Si oxide and OMCA |

### Examples 7A-D (Table 8)

In these examples, the standard formulation for O/W emulsions is used. The Zn-Si oxide particles from Example 1B are introduced into the oil phase of the system.

| | |
|---|---|
| A | standard formulation O/W emulsion with Zn-Si oxide |
| B | standard formulation O/W emulsion with OMC |
| C | standard formulation O/W emulsion with Zn-Si oxide and OMC |
| D | standard formulation O/W with Zn-Si oxide, OMC and isostearic acid |

### Examples 8A-C (Table 9)

In these examples, the standard formulation for W/O emulsions is used. The Zn-Si oxide particles from Example 1B are introduced into the oil phase of the system.

| | |
|---|---|
| A | standard formulation W/O emulsion with Zn-Si oxide |
| B | standard formulation W/O emulsion with PISA |
| C | standard formulation W/O emulsion with Zn-Si oxide and PISA |

### Examples 9A-D (Table 10)

In these examples, the standard formulation for O/W emulsions is used. The Zn-Si oxide particles from Example 1B are introduced into the oil phase of the system.

| | |
|---|---|
| A | standard formulation O/W emulsion with Zn-Si oxide |
| B | standard formulation O/W emulsion with PISA |
| C | standard formulation O/W emulsion with Zn-Si oxide and PISA |
| D | standard formulation O/W emulsion with Zn-Si oxide, PISA and isostearic acid |

### Examples 10A-C (Table 11)

In these examples, the standard formulation for W/O emulsions is used. The Zn-Si oxide particles from Example 1B are introduced into the oil phase of the system.

| | |
|---|---|
| A | standard formulation W/O emulsion with Zn-Si oxide |
| B | standard formulation W/O emulsion with BEMT |
| C | standard formulation W/O emulsion with Zn-Si oxide and BEMT |

### Examples 11A-D (Table 12)

In these examples, the standard formulation for O/W emulsions is used. The Zn-Si oxide particles from Example 1B are introduced into the oil phase of the system.

| | |
|---|---|
| A | standard formulation O/W emulsion with Zn-Si oxide |
| B | standard formulation O/W emulsion with BEMT |
| C | standard formulation O/W emulsion with Zn-Si oxide and BEMT |
| D | standard formulation O/W emulsion with Zn-Si oxide, BEMT and isostearic acid |

**Table 1: Feed materials and reaction conditions for the preparation of the zinc-silicon oxide particles used**

| **Example** | | **1A** | **2A** | **3A** |
|---|---|---|---|---|
| **Vaporization zone** | | | | |
| Zinc | kg/h | 3 | 4 | 4 |
| Hydrogen | Nm³/h | 14.5 | 14.5 | 16.2 |
| Air | Nm³/h | 30 | 30 | 34 |
| Nitrogen | Nm³/h | 15 | 15 | 14 |
| Lambda | | 0.80 | 0.78 | 0.80 |
| Temperature | °C | 1080 | 1050 | 1100 |
| Average residence time | ms | 1009 | 1026 | 923 |

| **Zn oxidation zone** | | | | |
|---|---|---|---|---|
| Air | Nm³/h | 65 | 80 | 80 |
| Lambda | | 8.76 | 9.71 | 9.59 |
| Temperature | °C | 800 | 820 | 920 |
| Average residence time | ms | 29 | 41 | 37 |

| **Si oxidation zone** | | | | |
|---|---|---|---|---|
| Si precursor | | Si(OEt)₄ | Si(OEt)₄ | Si(OEt)₄ |
| Si precursor | kg/h | 2.45 | 1.37 | 2.45 |
| Atomization air | Nm³/h | 4 | 4 | 4 |
| Lambda | | 4.04 | 8.88 | 4.96 |
| Temperature | °C | 760 | 610 | 820 |
| Average residence time | ms | 51 | 38 | 30 |

| **Quenching zone** | | | | |
|---|---|---|---|---|
| Air | Nm³/h | 200 | 200 | 300 |

**Table 2: Physicochemical properties of the zinc-silicon oxide particles used**

| **Example** | | **1A** | **2A** | **3A** |
|---|---|---|---|---|
| Zn/Si | at%/at% | 9.1 | 9.3 | 5.2 |
| Total of Zn+Si+O* | % | > 99.7 | > 99.7 | > 99.7 |
| BET surface area | m²/g | 35 | 29 | 25 |
| Weight-averaged primary particle diameter | nm | 23 | 24 | 35 |
| Average aggregate area | nm² | 1244 | 12 302 | 33 712 |
| Average aggregate diameter (ECD) | nm | 105 | 110 | 170 |
| Coating | nm | 2.1 | 1.2 | 2.3 |
| Carbon content | % by wt. | 0.15 | 0.15 | 0.1 |
| UV max. absorption | nm | 369 | 367 | 369 |
| Transparency | | 4.5 | 5.0 | 4.0 |

| | | | | |
|---|---|---|---|---|
| *) Calculated from ZnO and SiO₂, determined by X-ray fluorescence analysis; n.d. = not determined; | | | | |

**Table 3: Feed materials and adjustments for the preparation of the surface-modified zinc-silicon oxide particles**

| **Example** | | **1B** | **2B** | **3B** |
|---|---|---|---|---|
| Zinc oxide | | 1A | 2A | 3A |
| Silanizing agent | | Octyl-trimethoxy silane | Octyl-trimethoxy silane | Poly-dimethylsiloxane |
| Amount^{a)} | % by wt. | 1.5 | 3 | 2 |
| Amount of H₂O^{a)} | % by wt. | 0 | 0.2 | 0 |
| Temperature | °C | 140 | 140 | 160 |
| Time duration | h | 1.2 | 1.2 | 1.4 |

| | | | | |
|---|---|---|---|---|
| a) Based on 100 parts of Zn-Si oxide particles | | | | |

**Table 4: Surface-modified zinc-silicon oxide particles according to the invention**

| **Example** | | **1B** | **2B** | **3B** |
|---|---|---|---|---|
| BET surface area | m²/g | 33 | 28 | 25 |
| C content | % by wt. | 0.65 | 1.15 | 0.95 |
| Average aggregate area | nm² | 15 417 | 17 400 | 20 345 |
| Equivalent circle diameter of aggregate | nm | 86 | 120 | 140 |
| Average aggregate circumference | nm | 987 | 1006 | 1182 |

**Table 5: W/O formulations - Example 4 (in %)**

| **Phase** | **INCI** | **4A** | **4B** | **4C** |
|---|---|---|---|---|
| **A** | Cetyl PEG/PPG-10/1 dimethicone | 2.5 | 2.5 | 2.5 |
| | Ethylhexyl stearate | 12.5 | 12.5 | 10.0 |
| | Mineral oil | 12.5 | 12.5 | 10.0 |
| | Isostearic acid | 1.0 | 1.0 | 1.0 |
| | Hydrogenated castor oil | 0.5 | 0.5 | 0.5 |
| | Microcrystalline wax | 1.0 | 1.0 | 1.0 |
| | Octocrylene | | 5.0 | 5.0 |
| | Zn-Si oxide | 5.0 | | 5.0 |
| **B** | Sodium chloride | 0.5 | 0.5 | 0.5 |
| | Aqua | 64.45 | 64.45 | 64.45 |
| | 2-Bromo-2-nitropropane-1,3-diol | 0.05 | 0.05 | 0.05 |
| **SPF** | | 2 | 3 | 6 |

**Table 6: O/W formulations - Example 5 (in %)**

| **Phase** | **Constituents** | **5A** | **5B** | **5C** | **5D** |
|---|---|---|---|---|---|
| **A** | Ceteareth-15, glyceryl stearate | 2.5 | 2.5 | 2.5 | 2.5 |
| | Glyceryl stearate | 1.0 | 1.0 | 1.0 | 1.0 |
| | Stearyl alcohol | 2.0 | 2.0 | 2.0 | 2.0 |
| | C12-15 alkyl benzoate | 14.5 | 9.5 | 9.5 | 8.5 |
| | Octocrylene | | 5.0 | 5.0 | 5.0 |
| | Zn-Si oxide | 10.0 | | 10.0 | 10.0 |
| | Isostearic acid | | | | 1.0 |
| **B** | Glycerine | 3.0 | 3.0 | 3.0 | 3.0 |
| | Aqua | 66.5 | 76.5 | 66.5 | 66.5 |
| | Chloroacetamide | 0.1 | 0.1 | 0.1 | 0.1 |
| **C** | Xanthan gum | 0.4 | 0.4 | 0.4 | 0.4 |
| **SPF** | | 2 | 3 | 8 | 9 |

**Table 7: W/O formulations - Example 6 (in %)**

| **Phase** | **Constituents** | **6A** | **6B** | **6C** |
|---|---|---|---|---|
| **A** | Cetyl PEG/PPG-10/1 dimethicone | 2.5 | 2.5 | 2.5 |
| | Ethylhexyl stearate | 12.5 | 12.5 | 10.0 |
| | Mineral oil | 12.5 | 12.5 | 10.0 |
| | Isostearic acid | 1.0 | 1.0 | 1.0 |
| | Hydrogenated castor oil | 0.5 | 0.5 | 0.5 |
| | Microcrystalline wax | 1.0 | 1.0 | 1.0 |
| | Ethylhexyl methoxycinnamate | | 5.0 | 5.0 |
| | Zn-Si oxide | 5.0 | | 5.0 |
| **B** | Sodium chloride | 0.5 | 0.5 | 0.5 |
| | Aqua | 64.45 | 64.45 | 64.45 |
| | 2-Bromo-2-nitropropane-1,3-diol | 0.05 | 0.05 | 0.05 |
| **SPF** | | 2 | 7 | 13 |

**Table 8: O/W formulations - Example 7 (in %)**

| **Phase** | **Constituents** | **7A** | **7B** | **7C** | **7D** |
|---|---|---|---|---|---|
| **A** | Ceteareth-15, glyceryl stearate | 2.5 | 2.5 | 2.5 | 2.5 |
| | Glyceryl stearate | 1.0 | 1.0 | 1.0 | 1.0 |
| | Stearyl alcohol | 2.0 | 2.0 | 2.0 | 2.0 |
| | C12-15 alkyl benzoate | 14.5 | 9.5 | 9.5 | 8.5 |
| | Ethylhexyl methoxy-cinnamate | | 5.0 | 5.0 | 5.0 |
| | Zn-Si oxide | 10.0 | | 10.0 | 10.0 |
| | Isostearic acid | | | | 1.0 |
| **B** | Glycerine | 3.0 | 3.0 | 3.0 | 3.0 |
| | Aqua | 66.5 | 76.5 | 66.5 | 66.5 |
| | Chloroacetamide | 0.1 | 0.1 | 0.1 | 0.1 |
| **C** | Xanthan gum | 0.4 | 0.4 | 0.4 | 0.4 |
| **SPF** | | 2 | 6 | 11 | 16 |

**Table 9: W/O formulations - Example 8 (in %)**

| **Phase** | **Constituents** | **8A** | **8B** | **8C** |
|---|---|---|---|---|
| **A** | Cetyl PEG/PPG-10/1 dimethicone | 2.5 | 2.5 | 2.5 |
| | Ethylhexyl stearate | 12.5 | 15.0 | 12.5 |
| | Mineral oil | 12.5 | 15.0 | 12.5 |
| | Isostearic acid | 1.0 | 1.0 | 1.0 |
| | Hydrogenated castor oil | 0.5 | 0.5 | 0.5 |
| | Microcrystalline wax | 1.0 | 1.0 | 1.0 |
| | Zn-Si oxide | 5.0 | | 5.0 |
| **B** | Sodium chloride | 0.5 | 0.5 | 0.5 |
| | Aqua | 64.45 | 49.45 | 49.45 |
| | 2-Bromo-2-nitropropane-1,3-diol | 0.05 | 0.05 | 0.05 |
| | Phenylbenzimidazole-sulphonic acid (20% aqua) | | 15.0 | 15.0 |
| **SPF** | | 2 | 5 | 9 |

**Table 10: O/W formulations - Example 9 (in %)**

| **Phase** | **Constituents** | **9A** | **9B** | **9C** | **9D** |
|---|---|---|---|---|---|
| **A** | Ceteareth-15, glyceryl stearate | 2.5 | 2.5 | 2.5 | 2.5 |
| | Glyceryl stearate | 1.0 | 1.0 | 1.0 | 1.0 |
| | Stearyl alcohol | 2.0 | 2.0 | 2.0 | 2.0 |
| | C12-15 alkyl benzoate | 14.5 | 14.5 | 14.5 | 13.5 |
| | Zn-Si oxide | 10.0 | | 10.0 | 10.0 |
| | Isostearic acid | | | | 1.0 |
| **B** | Glycerine | 3.0 | 3.0 | 3.0 | 3.0 |
| | Aqua | 66.5 | 61.5 | 51.5 | 51.5 |
| | Chloroacetamide | 0.1 | 0.1 | 0.1 | 0.1 |
| | Phenylbenzimidazole-sulphonic acid (20% aqua) | | 15.0 | 15.0 | 15.0 |
| **C** | Xanthan gum | 0.4 | 0.4 | 0.4 | 0.4 |
| **SPF** | | 2 | 5 | 11 | 15 |

**Table 11: W/O formulations - Example 10 (in %)**

| **Phase** | **Constituents** | **10A** | **10B** | **10C** |
|---|---|---|---|---|
| **A** | Cetyl PEG/PPG-10/1 dimethicone | 2.5 | 2.5 | 2.5 |
| | C12-15 alkyl benzoate | 27.0 | 25.0 | 22.0 |
| | Isostearic acid | 1.0 | 1.0 | 1.0 |
| | Hydrogenated castor oil | 0.5 | 0.5 | 0.5 |
| | Microcrystalline wax | 1.0 | 1.0 | 1.0 |
| | Bis-ethylhexyloxyphenol-methoxyphenyl triazine | 3.0 | | 3.0 |
| | Zn-Si oxide | | 5.0 | 5.0 |
| **B** | Sodium chloride | 0.5 | 0.5 | 0.5 |
| | Aqua | 64.45 | 64.45 | 64.45 |
| | 2-Bromo-2-nitropropane-1,3-diol | 0.05 | 0.05 | 0.05 |
| **SPF** | | 2 | 8 | 13 |

**Table 12: O/W formulations - Example 11 (in %)**

| **Phase** | **Constituents** | **11A** | **11B** | **11C** | **11D** |
|---|---|---|---|---|---|
| **A** | Ceteareth-15, glyceryl stearate | 2.5 | 2.5 | 2.5 | 2.5 |
| | Glyceryl stearate | 1.0 | 1.0 | 1.0 | 1.0 |
| | Stearyl alcohol | 2.0 | 2.0 | 2.0 | 2.0 |
| | C12-15 alkyl benzoate | 14.5 | 12.5 | 12.5 | 11.5 |
| | Bis-ethylhexyloxyphenol-methoxyphenyl triazine | | 2.0 | 2.0 | 2.0 |
| | Zn-Si oxide | 10.0 | | 10.0 | 10.0 |
| | Isostearic acid | | | | 1.0 |
| **B** | Glycerine | 3.0 | 3.0 | 3.0 | 3.0 |
| | Aqua | 66.5 | 76.5 | 66.5 | 66.5 |
| | Chloroacetamide | 0.1 | 0.1 | 0.1 | 0.1 |
| **C** | Xanthan gum | 0.4 | 0.4 | 0.4 | 0.4 |
| **SPF** | | 2 | 3 | 6 | 8 |

## Claims

1. Surface-modified zinc-silicon oxide particles, **characterized in that**
- they consist of a core, a first coating layer surrounding the core, and a second coating layer surrounding the first coating layer, where
- the core is crystalline and consists of aggregated zinc oxide primary particles having a primary particle diameter of from 10 to 75 nm,
- the first coating layer consists of one or more compounds containing the elements Zn, Si and O,
- the second coating layer comprises linear and/or branched alkylsilyl groups having 1 to 20 carbon atoms bonded chemically to the first coating layer,
- they have an average aggregate area of less than 40 000 nm² and an average aggregate diameter (ECD) of less than 300 nm,
- they have a carbon content of from 0.4 to 1.5% by weight and
- they have a BET surface area of from 10 to 60 m²/g.

2. Surface-modified zinc-silicon oxide particles according to Claim 1, **characterized in that** the aggregates have an average, projected aggregate area of 8000-30 000 nm², an equivalent circle diameter (ECD) of 70-250 nm and an average circumference of 500-2000 nm.

3. Surface-modified zinc-silicon oxide particles according to Claim 1 or 2, **characterized in that** the proportion of lead is at most 20 ppm, of arsenic at most 3 ppm, of cadmium at most 15 ppm, of iron at most 200 ppm, of antimony at most 1 ppm and of mercury at most 1 ppm.

4. Surface-modified zinc-silicon oxide particles according to Claim 1, **characterized in that**
- the second coating layer consists of alkylsilyl groups having 1 to 8 carbon atoms,
- the carbon content is from 0.8 to 1.2% by weight,
- the BET surface area is 20 to 40 m²/g
- the average aggregate area is less than 20 000 nm² and the average aggregate diameter is less than 150 nm.

5. Process for the preparation of the surface-modified zinc-silicon oxide particles according to Claims 1 to 4, **characterized in that** zinc-silicon oxide particles are sprayed with one or more surface-modifying agents, optionally dissolved in an organic solvent, selected from the group comprising silanes, polysiloxanes and/or silazanes having in each case linear and/or branched C₁-C₂₀-alkyl units, and the mixture is then thermally treated at a temperature of from 120 to 200°C over a period of from 0.5 to 2 hours, optionally under protective gas, where the zinc-silicon oxide particles used
- have a Zn/Si ratio of from 2 to 75, in atom%/atom%,
- have a proportion of Zn, Si and O of at least 99% by weight, based on the zinc-silicon oxide particles,
- have a BET surface area of from 10 to 60 m²/g, a weight-averaged primary particle diameter of from 10 to 75 nm, an average aggregate area of less than 40 000 nm² and an average aggregate diameter (ECD) of less than 200 nm,
- consist of a crystalline core of aggregated primary particles of zinc oxide and of a coating which surrounds the aggregated zinc oxide primary particles and of one or more compounds containing the elements Si and O.

6. Process according to Claim 5, **characterized in that** the surface-modifying agent is selected from the group comprising
- the haloorganosilanes of the type X₃Si(CₙH₂ₙ₊₁), X₂(R')Si(CₙH₂ₙ₊₁), X(R')₂Si(CₙH₂ₙ₊₁), X₃Si(CH₂)ₘ-R', (R)X₂Si(CH₂)ₘ-R', (R)₂XSi(CH₂)ₘ-R' where X = Cl, Br; R = alkyl; R' = alkyl; n = 1-20; m = 1-20;
- the organosilanes of the type (RO)₃Si(CₙH₂ₙ₊₁), R'ₓ(RO)_{y}Si(CₙH₂ₙ₊₁), (RO)₃Si(CH₂)ₘ-R', (R")u(RO)vSi(CH₂)ₘ-R' where R = alkyl; R' = alkyl; n = 1-20; m = 1-20; x + y = 3; x = 1, 2; y = 1, 2; u + v = 2; u = 1, 2; v = 1, 2;
- silazanes of the type R'R₂Si-NH-SiR₂R' where R = alkyl, R' = alkyl, vinyl;
- polysiloxanes of the type 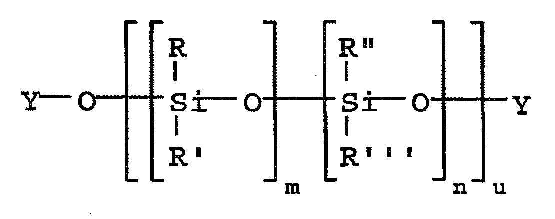 where R = alkyl, H; R' = alkyl, H; R" = alkyl, H; R"' = alkyl, H; Y = CH₃, H, CₚH₂ₚ₊₁ where p = 1-20; Y = Si(CH₃)₃, Si(CH₃)₂H, Si(CH₃)₂OH, Si(CH₃)₂(OCH₃), Si(CH₃)₂(CₚH₂ₚ₊₁) where p = 1-20; m = 0, 1, 2, 3, ...∞ ; n = 0, 1, 2, 3, ...∞ ; u = 0, 1, 2, 3, ...∞ or
- cyclic polysiloxanes of the type D3, D4 and/or D5.

7. Dispersion comprising the surface-modified zinc-silicon oxide particles according to Claims 1 to 4.

8. Sun protection formulation comprising the surface-modifying zinc-silicon oxide particles according to Claims 1 to 4 or the dispersion according to Claim 7.

## Patentansprüche

1. Oberflächenmodifizierte Zink-Silicium-Oxidpartikel,
**dadurch gekennzeichnet, dass**
- sie aus einem Kern, einer den Kern umgebenden ersten Hüllschicht und einer die erste Hüllschicht umgebenden zweiten Hüllschicht bestehen, wobei
- der Kern kristallin ist und aus aggregierten Zinkoxid-Primärpartikeln mit einem Primärpartikeldurchmesser von 10 bis 75 nm besteht,
- die erste Hüllschicht aus einer oder mehreren, die Elemente Zn, Si und O enthaltenden Verbindungen besteht,
- die zweite Hüllschicht chemisch an die erste Hüllschicht gebundene lineare und/oder verzweigte Alkylsilylgruppen mit 1 bis 20 Kohlenstoffatomen enthält,
- sie eine mittlere Aggregatfläche von kleiner als 40000 nm² und einen mittleren Aggregatdurchmesser (ECD) von kleiner als 300 nm,
- sie einen Kohlenstoffgehalt von 0,4 bis 1,5 Gew.-% und
- sie eine BET-Oberfläche von 10 bis 60 m²/g aufweisen.

2. Oberflächenmodifizierte Zink-Silicium-Oxidpartikel nach Anspruch 1, **dadurch gekennzeichnet, dass** die Aggregate eine mittlere, projizierte Aggregatfläche von 8000-30000 nm², einen äquivalenten Kreisdurchmesser (ECD) von 70-250 nm und einen mittleren Umfang von 500-2000 nm aufweisen.

3. Oberflächenmodifizierte Zink-Silicium-Oxidpartikel nach den Ansprüchen 1 oder 2, **dadurch gekennzeichnet, dass** der Anteil an Blei höchstens 20 ppm, an Arsen höchstens 3 ppm, an Cadmium höchstens 15 ppm, an Eisen höchstens 200 ppm, an Antimon höchstens 1 ppm und an Quecksilber höchstens 1 ppm beträgt.

4. Oberflächenmodifizierte Zink-Silicium-Oxidpartikel nach Anspruch 1, **dadurch gekennzeichnet, dass**
- die zweite Hüllschicht aus Alkylsilylgruppen mit 1 bis 8 Kohlenstoffatomen besteht,
- der Kohlenstoffgehalt von 0,8 bis 1,2 Gew.-% ist,
- die BET-Oberfläche 20 bis 40 m²/g ist,
- die mittlere Aggregatfläche kleiner als 20000 nm² und der mittlere Aggregatdurchmesser kleiner als 150 nm ist.

5. Verfahren zur Herstellung der oberflächenmodifizierten Zink-Silicium-Oxidpartikel gemäss den Ansprüchen 1 bis 4, **dadurch gekennzeichnet, dass** man Zink-Silicium-Oxidpartikel mit einem oder mehreren, gegebenenfalls in einem organischen Lösungsmittel gelösten, oberflächenmodifizierenden Mitteln, ausgewählt aus der Gruppe umfassend Silane, Polysiloxane und/oder Silazane mit jeweils linearen und/oder verzweigten C₁-C₂₀-Alkyleinheiten, besprüht und das Gemisch anschliessend bei einer Temperatur von 120 bis 200°C über einen Zeitraum von 0,5 bis 2 Stunden, gegebenenfalls unter Schutzgas, thermisch behandelt, wobei die eingesetzten Zink-Silicium-Oxidpartikel
- ein Verhältnis Zn/Si von 2 bis 75, in Atom%/Atom% aufweisen,
- einen Anteil von Zn, Si und O von wenigstens 99 Gew.-%, bezogen auf die Zink-Silicium-Oxidpartikel, aufweisen,
- eine BET-Oberfläche von 10 bis 60 m²/g, einen gewichtsgemittelten Primärpartikeldurchmesser von 10 bis 75 nm, eine mittlere Aggregatfläche von weniger als 40000 nm² und einen mittleren Aggregatdurchmesser (ECD) von weniger als 200 nm aufweisen,
- aus einem kristallinen Kern aus aggregierten Primärpartikeln von Zinkoxid und aus einer Hülle, die die aggregierten Zinkoxid-Primärpartikel umgibt und aus einer oder mehreren, die Elemente Si und O enthaltenden Verbindungen bestehen.

6. Verfahren nach Anspruch 5, **dadurch gekennzeichnet, dass** das oberflächenmodifizierende Mittel ausgewählt ist aus der Gruppe umfassend
- die Halogenorganosilane des Types X₃Si(CₙH₂ₙ₊₁), X₂(R')Si(CₙH₂ₙ₊₁), X(R')₂Si(CₙH₂ₙ₊₁), X₃Si(CH₂)ₘ-R' (R)X₂Si(CH₂)ₘ-R', (R)₂XSi(CH₂)ₘ-R' mit X=Cl, Br; R=Alkyl; R'=Alkyl; n = 1-20; m = 1-20;
- die Organosilane des Types (RO)₃Si(CₙH₂ₙ₊₁), R'ₓ(RO)_{y}Si(CₙH₂ₙ₊₁), (RO)₃Si(CH₂)ₘ-R', (R")u(RO)vSi(CH₂)ₘ-R' mit R=Alkyl; R'=Alkyl; n = 1-20; m = 1-20; x + y = 3; x = 1, 2; y = 1, 2; u + v = 2; u = 1, 2; v = 1, 2;
- Silazane des Types R'R₂Si-NH-SiR₂R' mit R=Alkyl, R'=Alkyl, Vinyl;
- Polysiloxane des Types 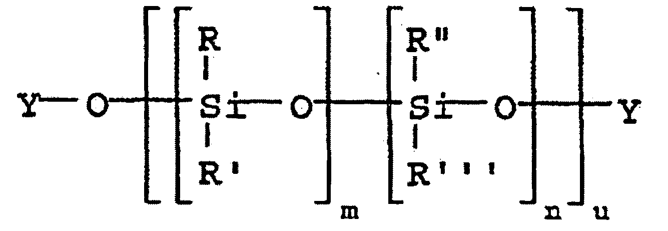 mit R=Alkyl, H; R'=Alkyl, H; R"=Alkyl, H; R"'=Alkyl, H; Y=CH₃, H, CₚH₂ₚ₊₁ mit p = 1-20; Y=Si(CH₃)₃, Si(CH₃)₂H, Si(CH₃)₂OH, Si(CH₃)₂(OCH₃), Si(CH₃)₂(CₚH₂ₚ₊₁) mit p = 1-20; m = 0, 1, 2, 3, ...∞; n = 0, 1, 2, 3, ...∞; u = 0, 1, 2, 3, ...∞ oder
- cyclische Polysiloxane des Types D3, D4 und/oder D5.

7. Dispersion enthaltend die oberflächenmodifizierten Zink-Silicium-Oxidpartikel gemäss den Ansprüchen 1 bis 4.

8. Sonnenschutzformulierung enthaltend die oberflächenmodifizierten Zink-Silicium-Oxidpartikel gemäss den Ansprüchen 1 bis 4 oder die Dispersion gemäss Anspruch 7.

## Revendications

1. Particules d'oxyde de zinc-silicium modifiées en surface, **caractérisées en ce que**
- elles se composent d'un coeur, d'une première couche de revêtement entourant le coeur, et d'une deuxième couche de revêtement entourant la première couche de revêtement, où
- le coeur est cristallin et consiste en des particules primaires agglomérées d'oxyde de zinc ayant un diamètre de particule primaire de 10 à 75 nm,
- la première couche de revêtement consiste en un ou plusieurs composés contenant les éléments Zn, Si et O,
- la deuxième couche de revêtement comprend des groupes alkylsilyle linéaires et/ou ramifiés portant 1 à 20 atomes de carbone liés chimiquement à la première couche de revêtement,
- elles ont une surface moyenne d'agrégat inférieure à 40 000 nm² et un diamètre moyen d'agrégat (ECD) inférieur à 300 nm,
- elles ont une teneur en carbone de 0,4 à 1,5 % en poids, et
- elles ont une surface spécifique BET de 10 à 60 m²/g.

2. Particules d'oxyde de zinc-silicium modifiées en surface selon la revendication 1, **caractérisées en ce que** les agrégats ont une surface projetée moyenne d'agrégat de 8000-30 000 nm², un diamètre de cercle équivalent (ECD) de 70-250 nm et une circonférence moyenne de 500-2000 nm.

3. Particules d'oxyde de zinc-silicium modifiées en surface selon la revendication 1 ou 2, **caractérisées en ce que** la proportion de plomb est au plus de 20 ppm, celle d'arsenic au plus de 3 ppm, celle de cadmium au plus de 15 ppm, celle de fer au plus de 200 ppm, celle d'antimoine au plus de 1 ppm, et celle de mercure au plus de 1 ppm.

4. Particules d'oxyde de zinc-silicium modifiées en surface selon la revendication 1, **caractérisées en ce que**
- la deuxième couche de revêtement consiste en des groupes alkylsilyle portant 1 à 8 atomes de carbone,
- la teneur en carbone est de 0,8 à 1,2 % en poids,
- la surface spécifique BET est de 20 à 40 m²/g,
- la surface moyenne d'agrégat est inférieure à 20 000 nm² et le diamètre moyen d'agrégat est inférieur à 150 nm.

5. Procédé pour la préparation des particules d'oxyde de zinc-silicium modifiées en surface selon les revendications 1 à 4, **caractérisé en ce que** des particules d'oxyde de zinc-silicium sont pulvérisées avec un ou plusieurs agents de modification de surface, éventuellement dissous dans un solvant organique, choisis dans le groupe comprenant les silanes, les polysiloxanes et/ou les silazanes ayant dans chaque cas des motifs alkyle en C₁-C₂₀ linéaires et/ou ramifiés, et le mélange est ensuite traité thermiquement à une température de 120 à 200°C sur une période de 0,5 à 2 heures, éventuellement sous gaz protecteur, où les particules d'oxyde de zinc-silicium utilisées
- ont un rapport Zn/Si de 2 à 75, en % atomique/% atomique,
- ont une proportion de Zn, Si et O d'au moins 99 % en poids, sur la base des particules d'oxyde de zinc-silicium,
- ont une surface spécifique BET de 10 à 60 m²/g, un diamètre moyen en poids de particule primaire de 10 à 75 nm, une surface moyenne d'agrégat inférieure à 40 000 nm² et un diamètre moyen d'agrégat (ECD) inférieur à 200 nm,
- se composent d'un coeur cristallin de particules primaires agglomérées d'oxyde de zinc et d'un revêtement qui entoure les particules primaires agglomérées d'oxyde de zinc et d'un ou plusieurs composés contenant les éléments Si et O.

6. Procédé selon la revendication 5, **caractérisé en ce que** l'agent de modification de surface est choisi dans le groupe comprenant
- les organosilanes halogénés du type X₃Si(CₙH₂ₙ₊₁), X₂(R')Si(CₙH₂ₙ₊₁), X(R')₂Si(CₙH₂ₙ₊₁), X₃Si(CH₂)ₘ-R', (R)X₂Si(CH₂)ₘ-R', (R)₂XSi(CH₂)ₘ-R', où X = Cl, Br ; R = radical alkyle ; R' = radical alkyle ; n = 1-20 ; m = 1-20 ;
- les organosilanes du type (RO)₃Si(CₙH₂ₙ₊₁), R'ₓ(RO)_{y}Si(CₙH₂ₙ₊₁), (RO)₃Si(CH₂)ₘ-R', (R")ᵤ(RO)ᵥSi(CH₂)ₘ-R', où R = radical alkyle ; R' = radical alkyle ; n = 1-20 ; m = 1-20 ; x + y = 3 ; x = 1, 2 ; y = 1, 2 ; u + v = 2 ; u = 1, 2 ; v = 1, 2 ;
- les silazanes du type R'R₂Si-NH-SiR₂R' où R = radical alkyle ; R' = radical alkyle, vinyle ;
- les polysiloxanes du type 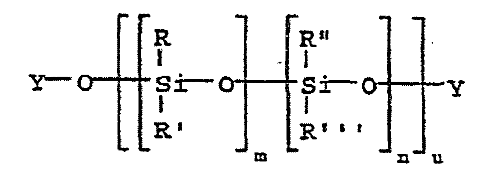 où R = radical alkyle, H ; R' = radical alkyle, H ; R" = radical alkyle, H ; R"' = radical alkyle, H ; Y = CH₃, H, CₚH₂ₚ₊₁ où p = 1-20 ; Y = Si(CH₃)₃, Si(CH₃)₂H, Si(CH₃)₂OH, Si(CH₃)₂(OCH₃), Si(CH₃)₂(CₚH₂ₚ₊₁) où p = 1-20 ; m = 0, 1, 2, 3, ... ∞ ; n = 0, 1, 2, 3, ... ∞ ; u = 0, 1, 2, 3, ... ∞ ; et
- les polysiloxanes cycliques du type D3, D4 et/ou D5.

7. Dispersion comprenant les particules d'oxyde de zinc-silicium modifiées en surface selon les revendications 1 à 4.

8. Formulation de protection solaire comprenant les particules d'oxyde de zinc-silicium modifiées en surface selon les revendications 1 à 4 ou la dispersion selon la revendication 7.
